# EUROPEAN PATENT SPECIFICATION

(11) **EP 1 267 902 B1**
(45) Date of publication and mention of the grant of the patent: **25.02.2004**
(21) Application number: 01915935.9
(22) Date of filing: 22.03.2001
(51) Int. Cl.: A61K 35/78, A61P 43/00, A61K 7/48

(54) **COSMETIC USE OF HOP AND ORNITHINE**
KOSMETISCHE VERWENDUNG VON HOPFEN UND ORNITHIN
UTILISATION COSMETIQUE DE HOUBLON ET D'ORNITHINE

(30) Priority: 24.03.2000 NL 1014751
(43) Date of publication of application: 02.01.2003
(73) Proprietor: Van de Wiel, Adriaan Emanuel Hendricus Anna Maria, 6718 TB Ede (NL); Christiaans, Marijke, 6718 TB Ede (NL)
(72) Inventor: Van de Wiel, Adriaan Emanuel Hendricus Anna Maria, 6718 TB Ede (NL); Christiaans, Marijke, 6718 TB Ede (NL)
(74) Representative: Visser-Luirink, Gesina, Dr.
(86) International application number: PCT/NL2001/000240
(87) International publication number: WO 2001/070249

(56) References cited:
- EP-A- 0 782 858
- "ERDIC" INTERNET, [Online] 15 June 2001 (2001-06-15), XP002169827 ERDIC Retrieved from the Internet: <URL:http://www.erdic.co.uk/ingredientsuk. htm> [retrieved on 2001-06-15]

## Description

The invention relates to a composition which contains at least hop and ornithine and which can be applied as cosmetic agent, in particular for enhancing growth and strengthening of breast tissue, or as medication for suppressing and preventing benign cysts, and possibly for suppressing malignant cancer cells.

Hop has for centuries been incorporated in food, including bread, by diverse peoples. This was done particularly to stimulate the production of mother's milk. Through the use of sufficient hop the volume of the mammary gland tissue increased, the breasts were enlarged, whereby the production of mother's milk increased. When the intake of hop is stopped the mammary gland tissue is reduced, whereby breast size decreases.

There is a great demand among the female part of the population for breast-enlarging means. Through the ages a prominent breast size has been for many women one of the ideal characteristics of beauty. Breast-enlargement operations are therefore very much a matter of course in plastic surgery and are even one of the most commonplace operations. For breast enlargement recourse is usually had to silicon implants, the drawbacks of which have since become known and which sometimes only manifest themselves in the medium or long term, quite apart from the discomfort of the operation itself.

The known growth agent of the type stated in the preamble does not have these drawbacks since no surgery whatever is involved and the active constituents of the agent consist, if not completely then for the major part, of wholly natural agricultural products. Hop, among other substances in the composition, is responsible for activating the mammary glands in the breast tissue, which then results in breast growth.

A substance containing hop with anti-growth action is known from J. Agric. Food Chem., 47 (1), 1999, and from the International patent application WO 00/53205. However, there continues to exist a need for other agents based on natural substances with a better action against benign cysts.

The known substance containing only hop is not ideal because the action is not strong and the result is not permanent.

The present invention has for its object to provide a cosmetic agent and/or medication of the type stated in the preamble with an enhanced action which thereby provides a greater and permanent result. In order to achieve the intended objective, a composition for enhancing breast growth and as medication which suppresses cysts of the type stated in the preamble has the feature according to the invention that the composition also contains omithine. This involves a balanced interaction between the different components of the composition. The hop present therein stimulates the mammary gland activity in the breast tissue, while omithine, and in particular L-ornithine, is as amino acid a cell-building substance and is responsible for a stronger development of the breast tissue. Ornithine has unexpectedly also been found to have a synergistic effect in causing benign cysts to subside and on the possible activity against cancerous growths. This composition further improves and stabilizes the condition of the female breast in the case of cysts and mammary gland infection. Another significant advantage compared to the action of hop alone is that the use of the combination of hop and omithine has a permanent effect, particularly when used to enhance growth and strengthening of the breast tissue.

The hop in the composition comprises numerous substances and trace elements which contribute toward breast growth, of which phytohormones such as genistein and daidzein are important examples. It is further assumed that the constituents of hop, genistein and daidzein, which belong to the phyto-oestrogenic substances or isoflavonoids, can have tumour-inhibiting action. Just as the body's own oestrogens, genistein can bond to the oestrogen receptor of the damaged cell, whereby further division is inhibited. Tumours which depend for their development on oestrogen such as that in breast tissue, the prostate and the intestines, would thus be inhibited in their growth. This theory is supported by a low death rate from breast cancer among women in Asia, which is generally attributed to the phyto-oestrogenic activity of isoflavonoids such as genistein and daidzein from soya, much more of which is consumed than in the western world.

In a preferred embodiment of the composition grain is added to hop and ornithine. Fibres from grain provide a better intestinal activity, whereby the components of the composition are more readily absorbed by the body. For the grain is therefore preferably used the full, unhusked grain, including skin. A significant enhancement of the action of the composition is thus obtained. A particular embodiment of the composition according to the invention has the feature that the grain type comprises at least one grain type from a group comprising buckwheat, rye, maize, wheat and barley, and more particularly barley. These grain types all contain to greater or lesser degree the substances which stimulate the intestines into increased activity, so that a more efficient metabolism is achieved.

An important preferred embodiment according to the invention has the feature that fennel or a derivative thereof is also present therein. A derivative of fennel is understood to mean (dried) fennel leaves, an extract of fennel leaves and in particular fennel seed. The inclusion hereof in the composition brings about a significantly improved action thereof. Fennel seed, like hop, contains phytohormones. These phytohormones complement the phytohormones of the hop. A further preferred embodiment of the composition according to the invention therefore has the feature that the fennel derivative comprises fennel seed.

Good results have been achieved with a particular embodiment of the composition according to the invention which is characterized in that at least an active composition therein comprises 25-40% by weight hop, 1-10% by weight fennel, 5-15% by weight buckwheat, 1-10% by weight rye, 20-35% by weight barley, 5-15% by weight malt and 5-15% by weight L-ornithine.

The composition according to the invention can be prepared in different forms, in liquid form as well as in dry form. A particularly practical embodiment of the composition has the feature however that the composition is embodied in capsule or tablet form, wherein one or more excipients are normally also used, such as for instance silicon oxide, calcium phosphate, magnesium stearate, oats or starch. This composition can optionally also be ingested in solution or as suspension.

The preferable point of departure in respect of the composition is purely natural products and components obtained from natural products. This does however have the drawback that the concentrations of active substances are sometimes low and that a large amount of the composition therefore has to be ingested to ensure sufficient effect. A particular embodiment of the composition has in this respect the feature that the composition in dry form is processed into tablets with a minimum of 80% by weight of active substance and for the rest an excipient, wherein the tablets preferably have a weight which does not exceed 850 milligrams. It has been established that a tablet under 850 milligrams can still be easily ingested, while a heavier tablet could on occasion result in indigestion problems. A maximum quantity of the composition is thus still administered per tablet in acceptable manner.

Also falling within the scope of the invention is the preparation of a solution or suspension acting in one or more of the following cases: for the purpose of growth or strengthening of breast tissue, or so as to prevent or to aid recovery from undesired lumps in the breast tissue, or to suppress or prevent benign cysts and the growth thereof or possibly of malignant cancer cells, wherein a tablet or capsule as described above is mixed with water to form such a solution or suspension.

The invention will now be further elucidated with reference to a number of embodiments:

### Example I

Per 1000 grams of active substance to be prepared, about 350 grams hop, 100 grams buckwheat, 60 grams rye, 270 grams barley and 150 grams malt are placed together in a container and ground to an homogeneous mixture. About 70 grams of L-ornithine is added hereto per 1000 grams of active substance to be prepared. This is the natural levorotatory variant of omithine which can be extracted from sugar beets. The whole is then well mixed and mixed in a ratio of 700:120 with an excipient consisting of silicon oxide, calcium phosphate and magnesium stearate. Tablets of 820 mg each are then formed from this mixture.

### Example II

Per 1000 grams of active substance to be prepared, about 330 grams hop, 100 grams buckwheat, 60 grams rye, 270 grams barley, 150 grams malt and 40 grams fennel are finely ground in a container and supplemented with 50 grams of L-ornithine. This composition is mixed in a ratio of 700:120 with a suitable excipient and processed into tablets of 800 mg each.

### Example III

Per 1000 grams of active substance to be prepared, about 465 grams hop, 50 grams buckwheat, 50 grams rye, 200 grams barley, 85 grams malt and 50 grams fennel are finely ground in a container and supplemented with 100 grams of L-ornithine. This composition is mixed in a ratio of 700:120 with a suitable excipient and processed into tablets of 500 milligrams each. The concentrations of hop, omithine and fennel in these tablets are significantly higher than in the foregoing embodiments, thereby enabling a smaller tablet size. This facilitates ingestion thereof. Preferably natural flavourings can optionally be added to conceal the strong hop taste in these tablets.
After an ingestion period of a month to several months, all compositions described in these examples displayed the first result in the form of an enhanced breast development, which continued the longer the composition is used until a maximal effect is obtained. The use of the composition can then be cut down and optionally stopped.

### Example IV

A group of women in the age-group of 20 to 35 years with breasts free of cysts were investigated in a study into the effects of ingesting ERDIC®, a preparation containing hop, L-ornithine, fennel, buckwheat, rye, malt, barley and excipients, on the growth and firmness of the breast. Half of these women used ERDIC® tablets as prescribed, i.e. 10-15 tablets a day together with at least 2 litres of water. The other half used a placebo in the same way. Enlargement and firmness of the breasts were determined in both subjective and objective manner, i.e. by means of measuring the breast size and estimating the elasticity and the slackness of the breast.
The result is shown in tables 1 and 2 below.

**Table 1**

| breast enlargement | after 2 months | after 4 months | after 6 months |
|---|---|---|---|
| ERDIC® | 30% | 50% | 60% |
| Placebo | 10% | 10% | 20% |

**Table 2**

| breast firmness | after 2 months | after 4 months | after 6 months |
|---|---|---|---|
| ERDIC® | 20% | 50% | 80% |
| Placebo | 0% | 10% | 20% |

It will be apparent from this data that in most of the women the ERDIC® preparation according to the invention has a clearly positive effect on the size and particularly the firmness of the breast.

### Example V

A group of women in the age-group of 38 to 52 years with benign cysts in their breast were investigated in a study into the effects of ingesting ERDIC® (described in example IV) on the development of these cysts. This development was monitored both subjectively (see table 3) and objectively using an ultrasonograph and a mammograph (see table 4). The months show the period of ingestion of ERDIC®.

**Table 3**

| | start | 1 month | 3 months | 6 months |
|---|---|---|---|---|
| cysts | 100% | 50% | 15% | 10% |
| less pain | 0% | 30% | 60% | 60% |
| disappeared | 0% | 20% | 25% | 30% |

**Table 4**

| | start | 6 months |
|---|---|---|
| worsening | | 5% |
| stationary | 100% | 65% |
| improvement | | 35% |

This study shows that the more subjective observations as shown in table 3 are confirmed by the objective ultrasonographic and mammographic observations as shown in table 4.

Although the invention has been further elucidated solely with reference to these five embodiments, it will be apparent that the invention is by no means limited thereto. On the contrary, many more variations are possible for a person with ordinary skill in the art within the scope of the invention. Instead of in tablet form, the composition can thus be used in other form, for instance liquid. The stated constituents and quantities thereof in the agents of the given examples can also be further added to and varied in order to further optimize the action of the composition.

## Claims

1. Composition at least containing hop and omithine for use as cosmetic agent or as medication.

2. Composition as claimed in claim 1, wherein a grain type is also present.

3. Composition as claimed in claim 1 or 2, wherein the weight ratio of hop to ornithine amounts to 1.5 to 10.

4. Composition as claimed in one or more of the foregoing claims, wherein fennel or a derivative of fennel is also present.

5. Composition as claimed in claim 4, wherein the derivative of fennel comprises fennel seed.

6. Composition as claimed in claim 4 or 5, wherein the weight ratio of hop to fennel amounts to 2 to 10.

7. Composition as claimed in one or more of the claims 2-6, wherein the grain type comprises at least one grain type from a group comprising buckwheat, rye, maize, wheat and barley.

8. Composition as claimed in claim 7, wherein the grain type comprises barley.

9. Composition as claimed in one or more of the foregoing claims, **characterized in that** at least the active composition therein comprises 25-40% by weight hop, 1-10% by weight fennel, 5-15% by weight buckwheat, 1-10% by weight rye, 20-35% by weight barley, 5-15% by weight malt and 5-15% by weight L-ornithine.

10. Composition as claimed in one or more of the foregoing claims, **characterized in that** at least the active part therein comprises only natural products and components obtained from natural products.

11. Composition as claimed in any of the foregoing claims, **characterized in that** the composition in dry form is processed into tablets with a minimum of 80% by weight of active substance and for the rest an excipient.

12. Composition as claimed in claim 11, **characterized in that** the tablets have a weight which does not exceed 850 milligrams.

13. Use of a composition as claimed in any of the foregoing claims for the preparation of a cosmetic agent for the purpose of growth and/or strengthening of breast tissue, or to prevent or to aid recovery from lumps in the breast tissue.

14. Use of a composition as claimed in any of the claims 1-12 for the preparation of a medication to suppress benign cysts and the growth thereof and possibly to suppress and prevent malignant cancer cells.

15. Use as claimed in claim 14 for the purpose of suppressing and preventing benign cysts and possibly suppressing and preventing malignant cancer cells in the female breast or in the prostate.

16. Tablet or capsule containing hop ornithine, fennel or a derivative thereof and optional excipients.

17. Tablet or capsule containing hop, ornithine, fennel or a derivative thereof, a grain type and optional excipients.

18. Method of preparing a solution or suspension for the purpose of preventing or aiding recovery from lumps in the breast tissue or for the purpose of growth or strengthening of breast tissue, or to suppress and prevent benign cysts and the growth thereof or possibly to suppress and prevent malignant cells, wherein a tablet or capsule as described in claim 16 or 17 is mixed with water.

## Patentansprüche

1. Zusammensetzung, die mindestens Hopfen und Ornithin enthält, zur Verwendung als kosmetisches Mittel oder zur medizinischen Behandlung.

2. Zusammensetzung nach Anspruch 1, worin auch eine Getreideart vorliegt.

3. Zusammensetzung nach Anspruch 1 oder 2, worin das Gewichtsverhältnis von Hopfen zu Ornithin bei 1,5 bis 10 liegt.

4. Zusammensetzung nach einem der vorstehenden Ansprüche, worin auch Fenchel oder ein Fenchelderivat vorliegt.

5. Zusammensetzung nach Anspruch 4, worin das Fenchelderivat Fenchelsamen enthält.

6. Zusammensetzung nach Anspruch 4 oder 5, worin das Gewichtsverhältnis von Hopfen zu Fenchel 2 bis 10 beträgt.

7. Zusammensetzung nach einem der Ansprüche 2 bis 6, worin die Getreideart mindestens eines der Getreide Buchweizen, Roggen, Mais, Weizen und Gerste enthält.

8. Zusammensetzung nach Anspruch 7, worin die Getreideart Gerste enthält.

9. Zusammensetzung nach einem oder mehreren der vorstehenden Ansprüche, **dadurch gekennzeichnet, dass** mindestens die aktive Zusammensetzung darin 25 bis 40 Gew.% Hopfen, 1 bis 10 Gew.% Fenchel, 5 bis 15 Gew.% Buchweizen, 1 bis 10 Gew.% Roggen, 20 bis 35 Gew.% Gerste, 5 bis 15 Gew.% Malz und 5 bis 15 Gew.% L-Ornithin enthält.

10. Zusammensetzung nach einem oder mehreren der vorstehenden Ansprüche, **dadurch gekennzeichnet, dass** mindestens der aktive Teil darin nur natürliche Produkte und aus natürlichen Produkten erhaltene Komponenten enthält.

11. Zusammensetzung nach einem der vorstehenden Ansprüche, **dadurch gekennzeichnet, dass** die Zusammensetzung in Trockenform zu Tabletten mit einem Minimum von 80 Gew.% aktiver Substanz und mit dem Rest an einem Träger verarbeitet wird.

12. Zusammensetzung nach Anspruch 11, **dadurch gekennzeichnet, dass** die Tabletten ein Gewicht aufweisen, das 850 Milligramm nicht überschreitet.

13. Verwendung einer Zusammensetzung nach einem der vorstehenden Ansprüche zur Herstellung eines kosmetischen Mittels zum Zweck des Wachstums und/oder der Stärkung von Brustgewebe oder zur Veränderung oder zur Heilungsunterstützung von Knoten in dem Brustgewebe.

14. Verwendung einer Zusammensetzung nach einem der Ansprüche 1 bis 12 zur Herstellung eines Arzneimittels zur Unterdrückung von gutartigen Zysten und ihres Wachstums und möglicherweise zur Unterdrückung und Verhinderung von bösartigen Krebszellen.

15. Verwendung nach Anspruch 14 zum Zweck der Unterdrückung und Verhinderung von gutartigen Zysten und möglicherweise zur Unterdrückung und Verhinderung von bösartigen Krebszellen in der weiblichen Brust oder in der Prostata.

16. Tablette oder Kapsel mit einem Gehalt an Hopfen, Ornithin, Fenchel oder einem Derivat hiervon und gegebenenfalls Trägerstoffen.

17. Tablette oder Kapsel mit einem Gehalt an Hopfen, Ornithin, Fenchel oder einem Derivat hiervon, einer Getreideart und gegebenenfalls Trägerstoffen.

18. Verfahren zum Herstellen einer Lösung oder Suspension zum Zweck des Verhinderns oder der Heilungsunterstützung von Knoten in dem Brustgewebe oder zum Zweck des Wachstums oder des Festigens von Brustgewebe oder zum Unterdrücken und Verhindern von gutartigen Zysten sowie des Wachstums hiervon oder möglicherweise zum Unterdrücken und Verhindern von bösartigen Zellen, wobei eine Tablette oder Kapsel gemäß Anspruch 16 oder 17 mit Wasser gemischt wird.

## Revendications

1. Composition contenant au moins du houblon et de l'omithine pour une utilisation comme agent cosmétique ou comme médicament.

2. Composition telle que revendiquée dans la revendication 1, dans laquelle un type de céréale est également présent.

3. Composition telle que revendiquée dans la revendication 1 ou 2, dans laquelle le rapport en poids du houblon sur l'ornithine est de 1,5 à 10.

4. Composition telle que revendiquée dans une ou plusieurs des revendications précédentes, dans laquelle du fenouil ou un dérivé de fenouil est aussi présent.

5. Composition telle que revendiquée dans la revendication 4, dans laquelle le dérivé de fenouil comprend des graines de fenouil.

6. Composition telle que revendiquée dans la revendication 4 ou 5, dans laquelle le rapport en poids du houblon sur le fenouil est de 2 à 10.

7. Composition telle que revendiquée dans une ou plusieurs des revendications 2-6, dans laquelle le type de céréale comprend au moins un type de céréale du groupe comprenant le sarrasin, le seigle, le maïs, le blé et l'orge.

8. Composition telle que revendiquée dans la revendication 7, dans laquelle le type de céréale comprend l'orge.

9. Composition telle que revendiquée dans une ou plusieurs des revendications précédentes, **caractérisée en ce qu'**au moins la composition active comprend 25-40 % en poids de houblon, 1-10 % en poids de fenouil, 5-15 % en poids de sarrasin, 1-10 % en poids de seigle, 20-35 % en poids d'orge, 5-15 % en poids de malt et 5-15 % en poids de L-omithine.

10. Composition telle que revendiquée dans une ou plusieurs des revendications précédentes, **caractérisée en ce qu'**au moins la partie active comprend uniquement des produits naturels et des composants obtenus de produits naturels.

11. Composition telle que revendiquée dans l'une des revendications précédentes, **caractérisée en ce que** la composition sous forme sèche est préparée en comprimés avec un minimum de 80 % en poids de substance active et pour le reste un excipient.

12. Composition telle que revendiquée dans la revendication 11, **caractérisée en ce que** les comprimés ont un poids qui n'excède pas 850 milligrammes.

13. Utilisation d'une composition telle que revendiquée dans l'une des revendications précédentes pour la préparation d'un agent cosmétique destiné à la croissance et/ou au renforcement du tissu du sein, ou pour prévenir ou aider la guérison de bosses dans le tissu du sein.

14. Utilisation d'une composition telle que revendiquée dans l'une des revendications 1-12 pour la préparation d'un médicament destiné à supprimer les kystes bénins et la croissance de ceux-ci et éventuellement à supprimer ou prévenir les cellules cancéreuses malignes.

15. Utilisation selon la revendication 14 dans le but de supprimer et prévenir les kystes bénins et éventuellement supprimer et prévenir les cellules cancéreuses malignes dans le sein des femmes ou dans la prostate.

16. Comprimé ou capsule contenant du houblon, de l'ornithine, du fenouil ou un dérivé de celui-ci, et des excipients facultatifs.

17. Comprimé ou capsule contenant du houblon, de l'ornithine, du fenouil ou un dérivé de celui-ci, un type de céréale et des excipients facultatifs.

18. Méthode de préparation d'une solution ou suspension destinée à prévenir ou aider la guérison de bosses dans le tissu du sein ou destinée à la croissance ou au renforcement des tissus du sein, ou à supprimer et prévenir les kystes bénins et la croissance de ceux-ci, ou éventuellement à supprimer et prévenir les cellules cancéreuses malignes, dans laquelle un comprimé ou une capsule tel que décrit dans la revendication 16 ou 17 est mélangé avec de l'eau.
